(19) Europäisches Patentamt
European Patent Office
Office européen des brevets

(11) Veröffentlichungsnummer : **0 037 948**
**B1**

(12) # EUROPÄISCHE PATENTSCHRIFT

(45) Veröffentlichungstag der Patentschrift : **20.04.83**

(51) Int. Cl.³ : **C 07 C115/00**// C07C43/29, C07C87/06

(21) Anmeldenummer : **81102382.9**

(22) Anmeldetag : **30.03.81**

(54) **Verfahren zur Herstellung von N,N-Dimethyl-N'-(2-brom-4-methyl-phenyl)-triazen.**

(30) Priorität : **12.04.80 DE 3014122**

(43) Veröffentlichungstag der Anmeldung : **21.10.81 Patentblatt 81/42**

(45) Bekanntmachung des Hinweises auf die Patenterteilung : **20.04.83 Patentblatt 83/16**

(84) Benannte Vertragsstaaten : **BE CH DE FR GB IT LI NL**

(56) Entgegenhaltungen :
**DE A 2 652 810**

**IL FARMACO, Ed. Scient. 29 (1974) M. MAZZA et al. : « Sull' attivita fitotossica di derivati triazenici » Seiten 129-148**

**ORGANIC SYNTHESES COLLECTIVE, Band I, 1946, 2. Ausgabe, Seiten 111-113 John Wiley & Sons New York, US**

**JUSTUS LIEBIG'S ANNALEN DER CHEMIE Band 253 (1886), Seiten 255-271**

(73) Patentinhaber : **BAYER AG**
**Zentralbereich Patente, Marken und Lizenzen**
**D-5090 Leverkusen 1, Bayerwerk (DE)**

(72) Erfinder : **Stölzer, Claus, Dr.**
**Siller Strasse 44**
**D-5600 Wuppertal 11 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 037 948 B1

Jouve, 18, rue St-Denis, 75001 Paris, France

Verfahren zur Herstellung von N,N-Dimethyl-N'-(2-brom-4-methyl-phenyl)-triazen

Die Erfindung betrifft ein neues Verfahren zur Herstellung des bekannten N,N-Dimethyl-N'-(2-brom-4-methyl-phenyl)-triazens.

Es ist bekannt, daß man N,N-Dialkyl-N'-aryl-triazene, wie z. B. N,N-Dimethyl-N'-(2-brom-4-methyl-phenyl)-triazen, erhält, wenn man von aromatischen Aminen abgeleitete Diazoniumsalze, wie z. B. 2-Brom-4-methyl-benzol-diazoniumchlorid, mit sekundären Aminen, wie z. B. Dimethylamin, umsetzt (vgl. Il Farmaco, Ed. Sci *29* (1974), 129-148 — Chem. Abstr. *81* (1974), 34417 d).

Die entsprechenden aromatischen Amine, von welchen sich die Diazoniumsalze ableiten, erhält man jedoch in einzelnen Fällen nur über mehrstufige Prozesse, wobei insgesamt unbefriedigende Ausbeuten erzielt werden. So wird z. B. 2-Brom-4-methyl-anilin hergestellt, indem man p-Toluidin mit Essigsäure acetyliert und dann mit Brom umsetzt, das Zwischenprodukt isoliert, durch Umsetzung mit Salzsäure verseift, das Hydrochlorid des Verseifungsproduktes isoliert und daraus mit Natronlauge 2-Brom-4-methyl-anilin freigesetzt. Die Gesamtausbeute liegt zwischen 60 und 67 % d. Th. (vgl. Org. Synthese Coll. Vol. I (1932), 111-113).

Es wurde nun ein Verfahren zur Herstellung von N,N-Dimethyl-N'-(2-brom-4-methyl-phenyl)-triazen der Formel I

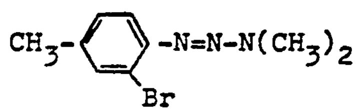

(I)

gefunden, indem man in einer 1. Stufe N-Acetyl-p-toluidin der Formel II

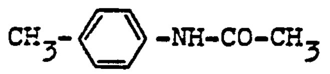

(II)

mit Brom, gegebenenfalls in Gegenwart eines Säureakzeptors und gegebenenfalls in Gegenwart eines Verdünnungsmittels bei Temperaturen zwischen 0 und 100 °C umsetzt, das Zwischenprodukt der Formel III

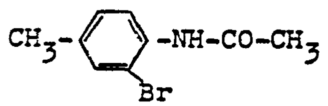

(III)

gegebenenfalls durch Zugabe von Wasser ausfällt und gegebenenfalls mit einem Reduktionsmittel behandelt, in einer 2. Stufe das isolierte und wieder gelöste Zwischenprodukt der Formel (III) mit verdünnter, wäßriger Salzsäure zum Hydrochlorid umsetzt und das isolierte und wieder gelöste Hydrochlorid auf Temperaturen zwischen 50 und 120 °C erhitzt, dann bei Temperaturen zwischen − 20 und + 30 °C mit einem Alkalinitrit behandelt und schließlich mit Dimethylamin, gegebenenfalls in Gegenwart eines Säurebindemittels, bei Temperaturen zwischen 0 und 50 °C umsetzt, das dadurch gekennzeichnet ist, daß man das Zwischenprodukt der Formel III

a) ohne es zuvor mit Wasser auszufällen in seiner Reaktionslösung der 1. Stufe mit verdünnter wässriger Salzsäure versetzt und ohne Isolierung des Hydrochlorids erhitzt, oder

b) aus seiner Reaktionslösung der 1. Stufe durch Vermischen mit Wasser ausfällt, abfiltriert und ohne Reinigung oder Trocknung mit verdünnter wässriger Salzsäure ohne Isolierung des Hydrochlorids erhitzt und

in beiden Fällen a) und b) das in der wäßrigen Lösung enthaltene Hydrochlorid von 2-Brom-4-methylanilin mit Alkalinitrit und Dimethylamin zum Triazen der Formel I umsetzt.

Es ist als überraschend anzusehen, daß nach dem erfindungsgemäßen Verfahren N,N-Dimethyl-N'-(2-brom-4-methyl-phenyl)-triazen mit hoher Ausbeute und guter Reinheit erhalten werden kann, da man normalerweise erwarten mußte, daß bei Überschlagen der bislang notwendigen Isolierungs- und Reinigungsschritte Ausbeuteverluste durch Nebenreaktionen erhalten werden und das Endprodukt stark verunreinigt ist.

Die bei dem erfindungsgemäßen Verfahren ablaufenden Reaktionen können durch das nachstehende Formelschema skizziert werden.

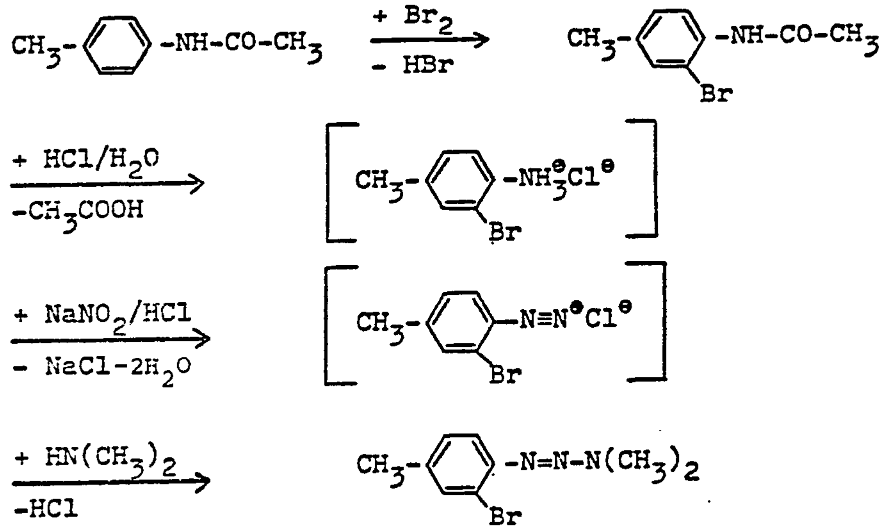


Das als Ausgangsstoff einzusetzende N-Acetyl-p-toluidin ist ein handelsübliches Produkt.

Das erfindungsgemäße Verfahren wird vorzugsweise in Gegenwart eines mit Wasser mischbaren, gegenüber Brom relativ inerten organischen Verdünnungsmittels durchgeführt. Besonders geeignet sind aliphatische Carbonsäuren, wie z. B. Ameisensäure, Essigsäure und Propionsäure. Essigsäure wird als Verdünnungsmittel für die erste Verfahrensstufe besonders bevorzugt. Zusätzlich wird gegebenenfalls Wasser als zweite Solvenskomponente verwendet. In der zweiten Stufe dient im allgemeinen Wasser als Lösungsmittel.

Als Säureakzeptoren können die üblichen Säurebindemittel verwendet werden. Besonders geeignet sind Alkalihydroxide, wie z. B. Natrium- und Kaliumhydroxid, Alkalicarbonate und -hydrogencarbonate, wie z. B. Natrium- und Kaliumcarbonat und -hydrogencarbonat, Alkaliacetate und -formiate, wie z. B. Natriumacetat und -formiat, sowie Ammoniak und Amine wie z. B. Triethylamin.

Die Reaktionstemperatur kann in der ersten Verfahrensstufe innerhalb eines größeren Bereiches variiert werden. Im allgemeinen arbeitet man zwischen 0 und 100 °C, vorzugsweise bei 10-80 °C. Das erfindungsgemäße Verfahren wird im allgemeinen bei Normaldruck durchgeführt.

Als Reduktionsmittel können Verbindungen verwendet werden, welche zur reduktiven Entfernung überschüssigen Broms geeignet sind. Als Beispiele hierfür seien Schwefeldioxid, schweflige Säure und deren Salze, wie z. B. Natrium- und Kaliumsulfit und -hydrogensulfit genannt.

Auf 1 Mol N-Acetyl-p-toluidin setzt man in der ersten Stufe zwischen 1 und 1,5 Mol, vorzugsweise zwischen 1,05 und 1,3 Mol Brom, in der zweiten Stufe zwischen 2 und 5 Mol, vorzugsweise zwischen 2,5 und 4 Mol, Chlorwasserstoff enthaltende Salzsäure, zwischen 0,9 und 1,2 Mol, vorzugsweise zwischen 0,95 und 1,1 Mol, Nitrit und zwischen 1 und 3 Mol, vorzugsweise zwischen 1,5 und 2 Mol, Dimethylamin ein.

In einer bevorzugten Ausführungsform des erfindungsgemäßen Verfahrens wird in der ersten Stufe N-Acetyl-p-toluidin, gegebenenfalls unter einer Inertgasatmosphäre, wie z. B. unter Stickstoff, in einem der oben angegebenen Lösungsmittel oder einem Gemisch derselben vorgelegt und es werden nacheinander eine der obengenannten Säureakzeptoren, gegebenenfalls in wäßriger Lösung und Brom langsam dazu gegeben.

Nach etwa einstündigem Rühren wird mit Wasser verdünnt und gegebenenfalls eines der angegebenen Reduktionsmittel bis zur Entfärbung der Reaktionsmischung dazugegeben. Das kristallin anfallende Produkt der Formel (III) wird durch Filtration abgetrennt, jedoch nicht weiter gereinigt oder getrocknet.

Zur Durchführung der zweiten Verfahrensstufe wird das noch feuchte Zwischenprodukt der Formel (III) mit verdünnter wäßriger Salzsäure, deren Chlorwasserstoffanteil zwischen 1 und 10 Gewichtsprozent liegt, einige Stunden lang auf Temperaturen zwischen 50 und 120 °C, vorzugsweise zwischen 80 und 110 °C erhitzt. Dann wird nach Abkühlen des Gemisches auf Temperaturen zwischen − 20 und + 30 °C, vorzugsweise zwischen − 10 und + 15 °C, eine gesättigte wäßrige Alkalinitritlösung (welche vorzugsweise Natriumnitrit enthält) langsam eindosiert und nach kurzem Nachrühren wird dieses Reaktionsgemisch zu einer wäßrigen Lösung von Dimethylamin und gegebenenfalls einem der obengenannten Säurebindemittel bei Temperaturen zwischen 0 und 50 °C, vorzugsweise zwischen 10 und 40 °C gegeben.

Nach etwa einstündigem Rühren wird das kristallin anfallende Produkt der Formel I durch Filtration isoliert.

Das nach dem erfindungsgemäßen Verfahren herzustellende N,N-Dimethyl-N'-(2-brom-4-methylphenyl)-triazen ist als phytotoxisches, d. h. herbizides Produkt bekannt (vgl. Il Farmaco, Ed. Sci. *29* (1974), 129-148 — Chem. Abstr. *81* (1974), 34417 d).

Die Verbindung der Formel (I) kann jedoch auch als Zwischenprodukt zur Herstellung von insektizid wirksamen Pyrethroiden verwendet werden :

Hierzu wird das Triazen mit Fluorwasserstoff bei Temperaturen zwischen − 20 und + 150 °C umgesetzt, das hierbei erhaltene 3-Brom-4-fluortoluol gegebenenfalls durch Destillation gereinigt und mit Natrium- oder Kaliumphenolat in Gegenwart von Kupfer oder einer katalytisch wirksamen Kupferverbindung, wie z. B. Kupferoxid und in Gegenwart eines Verdünnungsmittels, wie z. B. N,N-Dimethylacetamid bei Temperaturen zwischen 120 und 180 °C umgesetzt. Das auf diese Weise erhältliche 3-Phenoxy-4-fluortoluol, welches durch Destillation gereinigt werden kann, ist bereits als Zwischenprodukt für Pyrethroide bekannt (vgl. DE-OS 2 709 264).

Beispiel

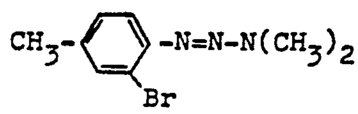

300 g Essigsäure werden in einem mit Stickstoff begasten Reaktionsgefäß vorgelegt und unter Rühren bei 20 °C mit 100 g N-Acetyl-p-toluidin portionsweise versetzt. Dann wird das Gemisch ca. 2 Stunden lang auf 50 °C erwärmt.

Anschließend werden nacheinander unter Rühren bei 50-55 °C 90 g 45 %ige Natronlauge und 130 g Brom langsam zum Reaktionsgemisch gegeben und nach beendeter Zugabe wird noch ca. 1 Stunde bei 50 °C nachgerührt.

Das Reaktionsgemisch wird dann mit 1 l Wasser verdünnt und mit einer gesättigten wäßrigen Lösung von 16 g Natriumhydrogensulfit versetzt. Vom auskristallisierten Zwischenprodukt wird abgesaugt und das noch feuchte Zwischenprodukt von der Nutsche in ein Reaktionsgefäß gegeben, in welches man zuvor 2 l Wasser eingefüllt hat. Das Gemisch wird unter Rühren auf 80 °C erwärmt und 233 g 30 %ige Salzsäure werden innerhalb von 30 Minuten eindosiert. Dann wird das Reaktionsgemisch 3 Stunden lang unter Rückfluß zum Sieden erhitzt.

Nach Abkühlen des Gemisches auf 0 °C gibt man eine gesättigte wäßrige Lösung von 45,5 g Natriumnitrit bei 0 bis 5 °C innerhalb 1 Stunde dazu und rühren dann ca. 30 Minuten bei dieser Temperatur nach. Das Reaktionsgemisch wird dann unter Rühren in ein anderes Reaktionsgefäß gegeben, in welchem man 222,5 g Natriumcarbonat und 77,5 g Dimethylamin in 640 ml Wasser vorgelegt hat und dessen Innentemperatur man bei 15-25 °C hält.

Nach einer weiteren Stunde Rühren, wird das Produkt durch Druckfiltration isoliert, dreimal mit je 800 ml Wasser gewaschen und bei 30 °C unter vermindertem Druck getrocknet.

Man erhält N,N-Dimethyl-N'-(2-brom-4-methyl-phenyl)-triazen in einer Ausbeute von 146 g (90 % der Theorie, bezogen auf eingesetzte N-Acetyl-p-toluidin).

**Anspruch**

Verfahren zur Herstellung von N,N-Dimethyl-N'-(2-brom-4-methyl-phenyl)-triazen der Formel I

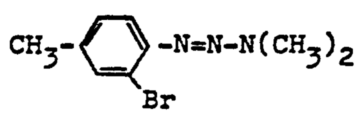

(I)

indem man in einer 1. Stufe N-Acetyl-p-toluidin der Formel II

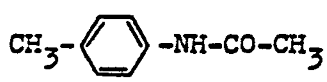

(II)

mit Brom, gegebenenfalls in Gegenwart eines Säureakzeptors und gegebenenfalls in Gegenwart eines Verdünnungsmittels bei Temperaturen zwischen 0 und 100 °C umsetzt, das Zwischenprodukt der Formel III

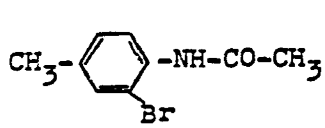

(III)

gegebenenfalls durch Zugabe von Wasser ausfällt und gegebenenfalls mit einem Reduktionsmittel behandelt, in einer 2. Stufe das isolierte und wieder gelöste Zwischenprodukt der Formel (III) mit verdünnter, wäßriger Salzsäure zum Hydrochlorid umsetzt und das isolierte und wieder gelöste Hydrochlorid auf Temperaturen zwischen 50 und 120 °C erhitzt, dann bei Temperaturen zwischen − 20 und + 30 °C mit einem Alkalinitrit behandelt und schließlich mit Dimethylamin, gegebenenfalls in Gegenwart eines Säurebindemittels, bei Temperaturen zwischen 0 und 50 °C umsetzt, das dadurch gekennzeichnet ist, daß man das Zwischenprodukt der Formel (III)

a) ohne es zuvor mit Wasser auszufällen, in seiner Reaktionslösung der 1. Stufe mit verdünnter wässriger Salzsäure versetzt und ohne Isolierung des Hydrochlorids erhitzt, oder

b) aus seiner Reaktionslösung der 1. Stufe durch Vermischen mit Wasser ausfällt, abfiltriert und ohne Reinigung oder Trocknung mit verdünnter wässriger Salzsäure ohne Isolierung des Hydrochlorids erhitzt und

in beiden Fällen a) und b) das in der wässrigen Lösung enthaltene Hydrochlorid von 2-Brom-4-methylanilin mit Alkalinitrit und Dimethylamin zum Triazen der Formel (I) umsetzt.

**Claim**

Process for the preparation of N,N-dimethyl-N'-(2-bromo-4-methyl-phenyl)-triazene of the formula I

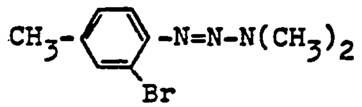
(I)

in which, in a 1st stage, N-acetyl-p-toluidine of the formula II

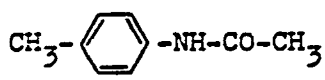
(II)

is reacted with bromine, if appropriate in the presence of an acid acceptor and if appropriate in the presence of a diluent, at temperatures between 0 and 100 °C, the intermediate product of the formula III

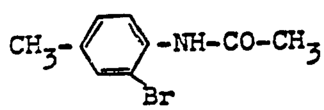
(III)

is, if appropriate, precipitated by adding water, and, if appropriate, is treated with a reducing agent, in a 2nd stage the isolated and re-dissolved intermediate product of the formula (III) is reacted with dilute, aqueous hydrochloric acid to produce the hydrochloride and the isolated and redissolved hydrochloride is heated to temperatures between 50 and 120 °C, is then treated with an alkali metal nitrite at temperatures between − 20 and + 30 °C and, finally, the product is reacted with dimethylamine, if appropriate in the presence of an acid-binding agent, at temperatures between 0 and 50 °C, which is characterised in that

a) without previously precipitating the intermediate product of the formula (III) with water dilute aqueous hydrochloric acid is added to said intermediate product in its reaction solution of the 1st stage and the mixture is heated without isolating the hydrochloride, or

b) the intermediate product of the formula (III) is precipitated from its reaction solution of the 1st stage by mixing with water, is filtered off and is heated, without being purified or dried, with dilute aqueous hydrochloric acid without isolating the hydrochloride and

in both cases a) and b) the hydrochloride of 2-bromo-4-methyl-aniline contained in the aqueous solution is reacted with an alkali metal nitrite and dimethylamine to give the triazene of the formula (I).

**Revendication**

Procédé de préparation du N,N-diméthyl-N'-(2-bromo-4-méthyl-phényl)-triazène de formule I

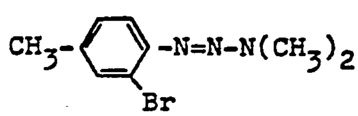
(I)

dans lequel, dans un premier stade opératoire, on fait réagir la N-acétyl-p-toluidine de formule II

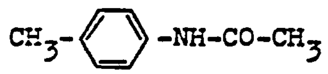

(II)

avec le brome, éventuellement en présence d'un accepteur d'acide et le cas échéant en présence d'un diluant à des températures de 0 à 100 °C, on précipite le produit intermédiaire de formule III

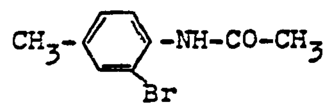

(III)

éventuellement par addition d'eau et on le traite le cas échéant par un agent réducteur, on convertit dans un deuxième stade le produit intermédiaire de formule III isolé et redissous, par l'acide chlorhydrique aqueux dilué, en le chlorhydrate et on chauffe le chlorhydrate isolé et redissous à des températures de 50 à 120 °C puis on le traite à des températures de − 20 à + 30 °C par un nitrite alcalin et finalement on fait réagir avec la diméthylamine, éventuellement en présence d'un agent fixant les acides, à des températures de 0 à 50 °C, ce procédé se caractérisant en ce que le produit intermédiaire de formule III

a) est additionné d'acide chlorhydrique aqueux dilué dans sa solution de réaction du premier stade sans avoir été précipité au préalable par l'eau, et le chlorhydrate est chauffé sans avoir été isolé, ou bien

b) est précipité de sa solution de réaction du premier stade par mélange avec de l'eau, filtré et chauffé sans purification ni séchage avec de l'acide chlorhydrique aqueux dilué, sans isolement du chlorhydrate, et

dans les deux cas a) et b), on fait réagir le chlorhydrate de la 2-bromo-4-méthylaniline contenu dans la solution aqueuse avec un nitrite alcalin et la diméthylamine, formant ainsi le triazène de formule I.